# EUROPEAN PATENT APPLICATION

(11) **EP 3 858 426 A1**
(43) Date of publication of application: **04.08.2021**
(21) Application number: 20305073.7
(22) Date of filing: 28.01.2020
(51) Int. Cl.: A61N 1/08, H01L 27/06, H01L 27/07, H01L 27/12, H01L 21/762, H01L 23/552, A61N 1/37, H01L 27/02, H01L 29/66

(54) **BIOLOGICAL-ELECTRODE PROTECTION MODULES AND THEIR FABRICATION METHODS**

(71) Applicant: Murata Manufacturing Co., Ltd., Nagaokakyo-shi, Kyoto 617-8555 (JP)
(72) Inventor: NORMAND, Nicolas, 14200 HEROUVILLE SAINT CLAIR (FR); VOIRON, Frédéric, 38530 BARRAUX (FR)
(74) Representative: Cabinet Beau de Loménie

(57) **Abstract**

A biological-electrode protection module (20) is a monolithic component comprising a capacitor (22) and a voltage-limiting component (24) integrated in a common substrate (25). The voltage-limiting component (24) has a low breakdown voltage no greater than 6 volts and may be a biphasic device operating in the punch-through mode. The protection module (20) is connected to or integrated with a set of biological electrodes (1) at a distance no greater than 1 cm. The capacitor may be a 3D capacitor, and common fabrication processes may be uses in forming the voltage-limiting component (24) and the capacitor (22). A JFET may be integrated in the same substrate (25) so that an electrical signal output from the monolithic protection device (20) is already pre-amplified.

## Description

### Field of the Invention

The present invention relates to the field of biological sensing/stimulation electronics. More particularly, the invention relates to biological-electrode protection modules and to methods of fabricating such modules.

### Technical Background

Various technologies have been developed to sense electrical biosignals in living beings. Thus, for example, various devices have been developed to measure electrical biosignals such as EECs (electroencephalograms), EMGs (electromyograms), ECGs (electrocardiograms), etc. Moreover, various devices have been developed to apply electrical signals to various body parts, for instance to perform deep brain stimulation, to stimulate cells in the spinal cord as a clinical therapy, etc. In general, biological electrodes are applied to the body so to enable the sensing/stimulation to be performed. In some applications it is desirable to be able to implant one or more biological electrodes within the body.

Typically, very small electrical signals (mV or µV) are measured using biological electrodes, and the measurement channel has high impedance (100KOhms, or even MOhms). Accordingly, the electrical signals output by biological electrodes tend to be extremely sensitive to interference and can easily be degraded by noise. The design of the signal acquisition electronics can have an impact on the accuracy with which the electrical biosignal can be measured.

The electrical signals from biological electrodes are generally handled using a small acquisition chain containing the following elements:
electrode ->protection module-> instrumentation amplifier (preamplifier) -> filtering module-> amplifiers -> sampling module -> software analysis module

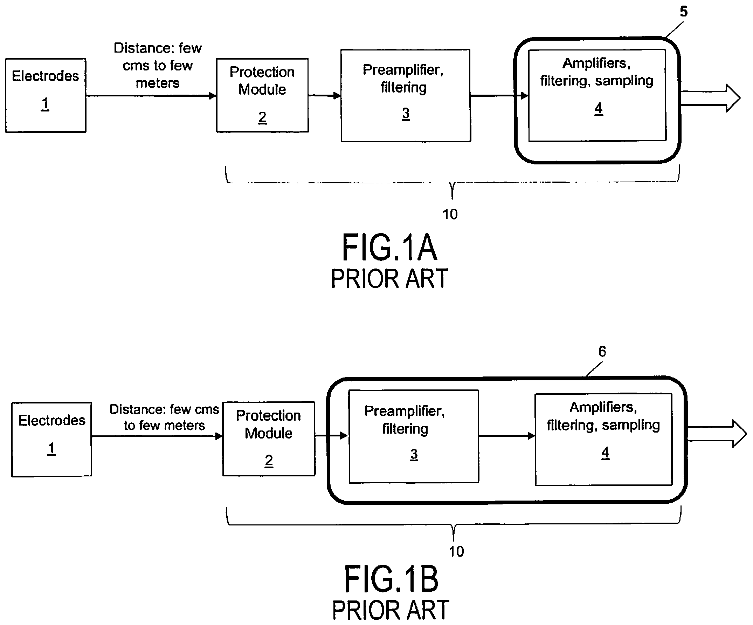

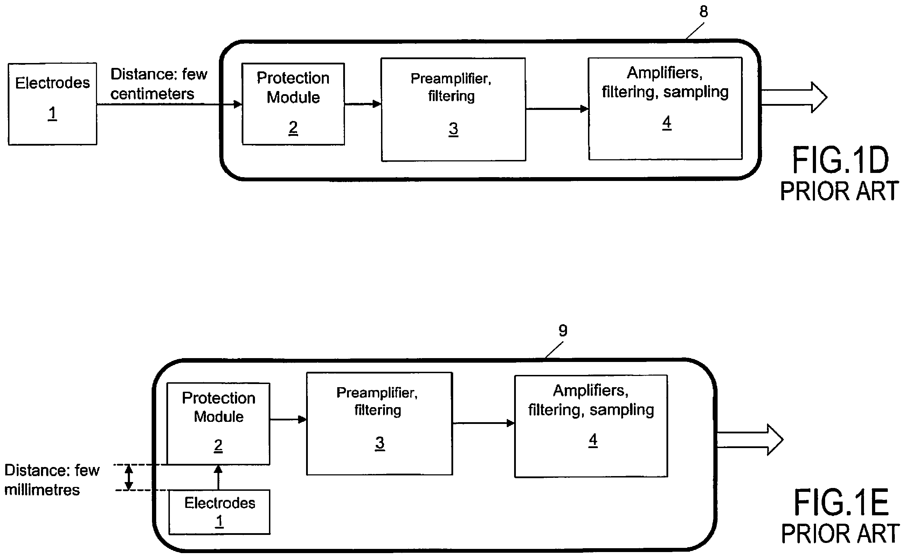

Various known electrical biosignal acquisition chains are illustrated in Figs. 1A to 1E.

Fig.1A illustrates a conventional configuration in which the biological electrodes 1 are spaced from a circuit board 10 which carries the electronics modules of the signal acquisition chain. Typically, the circuit board 10 has a surface area of some 10s of mm². The distance between the electrodes 1 and the circuit board 10 can be from a few centimetres to some meters. The very small electrical biosignal carried on the cables between the electrodes and the circuit board 10 is easily perturbed, for example by interference from mobile phone signals and power supply signals. Accordingly, the cable on which the electrical biosignal is carried may need to be implemented as a shielded cable.

Biological electrodes come in various sizes and shapes and are made from various materials. These electrodes are internal to the body (i.e. in contact with tissues, organs, cell systems, etc.) or external to the body (notably in contact with the skin). The electrodes 1 are made of biocompatible material. The characteristics of the electrodes are highly significant to the design of the acquisition chain. The electrical characteristic of interest (e.g. representing the sensed parameter, or the target characteristic of the stimulation signal) may be current, voltage, frequency and/or a specific waveform. The biological electrodes 1 are often provided as a set of electrodes on a cable or braid.

The electronics modules on the circuit board 10 include a protection module 1, a combined pre-amplifier and filtering module 2, and an ASIC (Application Specific Integrated Circuit) 5 combining components 4 for further amplification, filtering and sampling.

The protection module 2 contains discrete passive components, such as resistors, capacitors (nF range) and diodes. These elements are combined to form analogue filtering functions or security functions, for instance diodes are used as over-stress voltage suppressors to protect sensitive electronics from external unwanted electrical surges such as may arise in the case where the patient undergoes an MRI or is being operated on using an electrosurgical device. Capacitors are used as a DC blocking component to prevent any continuous voltage being applied to the body.

In the configuration illustrated in Fig.1A the instrumentation amplifier in the module 3 is a high-performance amplifier adapted to amplify the small signal produced by the electrodes 1. Typically, advanced technologies are required in order to produce suitable components to function as the instrumentation amplifier in view of the very small electrical signal received as input. The filtering components in the module 3 are adapted to extract the measurement signal from noise and/or interference that may be affecting it. Once again, advanced technologies may be required in order to produce suitably sophisticated filtering components. The elements 4 for further amplification, filtering and sampling can be general purpose amplifiers, filters and samplers because the electrical signal they are handling has already been amplified and therefore is less susceptible to noise/interference than the signal output by the electrodes 1. The output from the ASIC 5 is supplied to a software analysis module (not shown) to produce biological/medical data, e.g. an EEG, ECG, etc.

Fig.1B illustrates a variant of the Fig.1A type signal acquisition chain. In this variant, an ASIC 6 includes the instrumentation amplifier and filtering components 3, as well as the additional amplifier, filtering and sampling elements 4. Again, the circuit board 10 has a surface area of some 10s of mm², and the distance between the electrodes 1 and the circuit board 10 is from a few centimetres to some meters.

In configurations such as those of Figs.1A and 1B where an ASIC is used, care must be taken in designing the ASIC in order to maintain a high-quality signal in a reduced space. Most of the time the ASIC 5, 6 is far from the electrodes 1 and it becomes complicated to amplify the electrical signal from the electrodes given that the signal is subject to EMI interference. As a consequence, to get rid of parasitic signals there is an increase in the number of components used, for instance differential pairs may be used, and filtering low frequency parasitic signals requires large components and these are not the same as those requirements for filtering GSM signals. Accordingly, cost becomes high.

Furthermore, typically the ASIC has to perform impedance conversion between a high-impedance environment on the side of the biological electrodes and a low-impedance environment on the side of the software analysis module. This induces some limitations in terms of signal treatment, and signal acquisition will not be optimum. Therefore, the size and the power consumption of the ASIC increases, as well as the price.

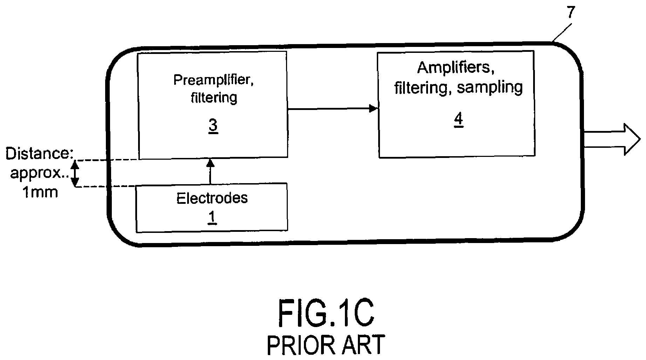

Fig.1C illustrates a configuration that has been proposed to sense biological electrical signals using a single advanced ASIC 7 (a so-called "lab on a chip") in the signal acquisition chain. According to this approach, the single advanced ASIC 7 contains the electrodes 1 as well as the instrumentation and filtering components 3, and the further amplifiers, filtering and sampling components 4. The ASIC 7 is an advanced ASIC in the sense that it is fabricated using advanced technologies and has a small size (around 10mm² surface area and low thickness). This configuration lacks a protection module and so does not prevent DC from being applied to a patient, nor does it prevent electrical surges from damaging the electronics. This configuration is not compliant with regulations relating to implantable devices, such as those of the US Food and Drug Administration.

Other proposals have been made to employ a SIP (System-in-Package) in the acquisition chain of biological electrical signals, in which various dies are assembled together in a common package. Fig.1D illustrates a first SIP-based approach as proposed in US 2016/0317820. In this configuration, a SIP 8 contains the protection module 2, the instrumentation and filtering components 3, and the further amplifiers, filtering and sampling components 4. The distance between the SIP and the electrodes is of the order of a few centimetres. The SIP is small, having a volume of the order of 1mm³. However, the very small signal from the electrodes can easily be perturbed, e.g. by interference.

Fig.IE illustrates a second SIP-based approach as proposed in US 2014/128937. In this configuration, an advanced SIP 9 contains the electrodes as well as the protection module 2, the instrumentation and filtering components 3, and the further amplifiers, filtering and sampling components 4. In this case, the distance between the SIP and the electrodes is of the order of a few millimetres. This configuration is biocompatible. The surface area of the advanced SIP 8 is 200mm², but the advanced SIP 8 is relatively thick (of the order of 10 mm).

A disadvantage of the SIP configurations, in addition to the fact that they tend to be expensive, is that manufacture of the SIP generally requires assembling together components that have been manufactured according to different technologies. This means that a large number of manufacturing steps are involved in producing the overall SIP. Moreover, as each technology type has its own failure modes, the concatenation of different technologies results in a large number of potential sources of failure. Furthermore, additional potential sources of failure result from the interconnections that must be made between the various SIP components.

The present invention has been made in the light of the above problems.

### Summary of the Invention

The present invention provides a biological-electrode protection module, comprising: a capacitor component and a voltage-limiting component integrated in a common substrate; and input and output terminals; wherein the voltage-limiting component has a breakdown voltage equal to or less than 6 volts, and one of the input and output terminals is configured to connect to each of a set of one or more biological electrodes.

By integrating the capacitor component and the low-breakdown-voltage voltage-limiting component in a common substrate, the protection device can be compact and thus it becomes easier to locate the protection module close to the biological electrodes, e.g. at a distance of 1 cm or less, or even to integrate the protection module with the biological electrodes as a kind of biological interface. In contrast to configurations that use ASIC technology, this platform is cost-effective and not area consuming.

In the present description, the breakdown voltage may be a reversible breakdown voltage.

In the biological-electrode protection module, the voltage-limiting component may be a component designed to have a breakdown voltage equal to or less than 3.3 volts, so that the biological-electrode protection module may be implanted into the human or animal body.

In the biological-electrode protection module the voltage-limiting component may be a biphasic device. In this way, the voltage-limiting component provides protection irrespective of the polarity of a voltage surge that may occur in the patient's body.

In some embodiments of biological-electrode protection modules according to the invention a pre-amplifier component is integrated into the same substrate as the capacitor component and the voltage-limiting component. Accordingly, the electrical signal output by the module has a larger amplitude and is less susceptible to degradation by noise/interference. This makes it possible to dispense with the need for advanced amplification and filtering components in the downstream part of the signal acquisition chain.

Such a preamplifier component may be implemented as a junction field effect transistor in the same substrate as the capacitor component and voltage-limiting component. The voltage-limiting component may be designed to operate in a punch-through mode (e.g. by being configured as a preferentially vertical bipolar structure of either type PNP or NPN).

The capacitor component may be a three-dimensional capacitor (i.e. a capacitor in which the electrodes and dielectric are contoured, for example by being formed conformally in wells in the substrate or conformally over columns/pillars in the substrate). This enables common technologies and process steps to be used during fabrication of the capacitor component, voltage-limiting component and pre-amplifier, reducing the cost of manufacture and reducing potential failure modes of the finished product.

The capacitor component may comprise plural individual capacitors that are electrically isolated from one another, for example, one capacitor for each biological electrode to which the protection module is to be connected bearing in mind that each biological electrode may correspond to a separate sensing and/or stimulation channel.

There may be isolation trenches, filled with electrically-insulating material, provided in the substrate to electrically isolate from each other the various electrical components formed in the substrate. In some embodiments the isolation trenches may be deep trenches (e.g. extending through substantially the whole thickness of the substrate) and may be formed in a common process with relief features (wells or columns/pillars) over which capacitor layers are to be formed. An integrated biological-electrode protection module according to the invention, incorporating such isolation trenches, provides excellent isolation between the sensing/stimulation channels. Moreover, in the case where the protection module incorporates a pre-amplifier component as well as the capacitor and voltage-limiting components, there can be superior rejection between adjacent channels in the cable interconnecting the protection module to the rest of the signal acquisition electronics. These two effects may make it possible to increase the overall number of channels included in the sensing/stimulation system and/or may allow the use of sensing and stimulation signals in very closely-spaced channels.

The present invention further provides a biological implant comprising a biological-electrode protection module as disclosed in the present document.

A biological implant incorporating a biological-electrode protection module according to the present disclosure can have a small size and yet provide sufficient protection to the body in which the device is implanted, especially in the case where the capacitor component is implemented as one or more three-dimensional capacitors (which can provide a large capacitance value in a small space). Moreover, by incorporating a voltage-limiting component having a low breakdown voltage, notably equal to or less than 6 volts, an adequate degree of protection can be assured for electronics modules connected to the implant.

The present invention still further provides a method of fabricating a biological-electrode protection module, comprising forming a capacitor component and a voltage-limiting component in a common substrate; and forming input and output terminals of the biological-electrode protection module; wherein the voltage-limiting component has a breakdown voltage equal to or less than 6 volts, and one of the input and output terminals is configured to connect to each of a set of one or more biological electrodes.

The fabrication method may include forming a pre-amplifier component in the substrate and common masking and doping steps may be used during the formation of the voltage-limiting component and the pre-amplifier component.

In the above-mentioned method a common process, such as an etching process, may form relief features (e.g. wells/holes/trenches or pillars/columns) in the substrate and may form one or more isolation trenches in the substrate to isolate the voltage-limiting and capacitor components (and pre-amplifier, if present) in the substrate from one another. The method may then further include providing electrically-insulating material in the isolation trench (es).

In the above-described fabrication method the forming of the voltage-limiting component may comprise forming a bipolar structure (preferably NPN) to create a voltage-limiting component that operates in punch-through mode, and the forming of the pre-amplifier component may comprises forming a junction field effect transistor. In this case a common set of process steps may be used in the formation of the voltage-limiting component, capacitor component and pre-amplifier component in the common substrate, avoiding the need for specific assembly steps to bring the components together. This reduces the potential failure modes of the finished module, leading to improved manufacturing yield.

### Brief Description of the Drawings

Further features and advantages of the present invention will become apparent from the following description of certain embodiments thereof, given by way of illustration only, not limitation, with reference to the accompanying drawings in which:
Figs 1A to 1E are block diagrams schematically representing known electrical-biosignal acquisition chains, in which:
   Fig.1A illustrates a first conventional approach using an ASIC,
   Fig.1B illustrates a variant of the conventional approach using an ASIC,
   Fig.1C illustrates an approach using an advanced ASIC,
   Fig.1D illustrates an approach using a SIP, and
   Fig.IE illustrates an approach using an advanced SIP;
Figs 2A and 2B are block diagrams schematically representing electrical-biosignal acquisition chains employing biological-electrode protection modules according to embodiments of the invention, in which:

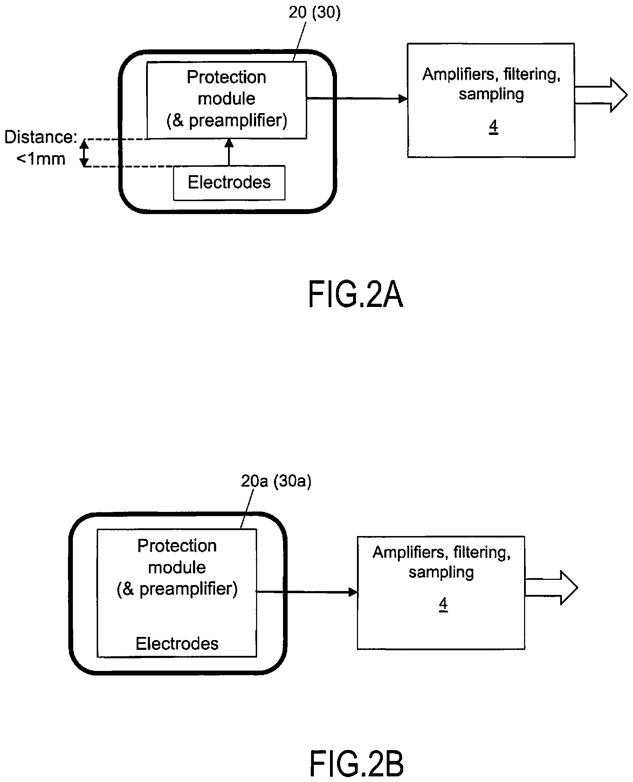

   Fig.2A illustrates a first arrangement in which a monolithic biological-electrode protection module according to an embodiment of the invention is connected to a set of biological electrodes,
   Fig.2B illustrates a first arrangement in which a biological-electrode protection module according to an embodiment of the invention is integrated with a set of biological electrodes;

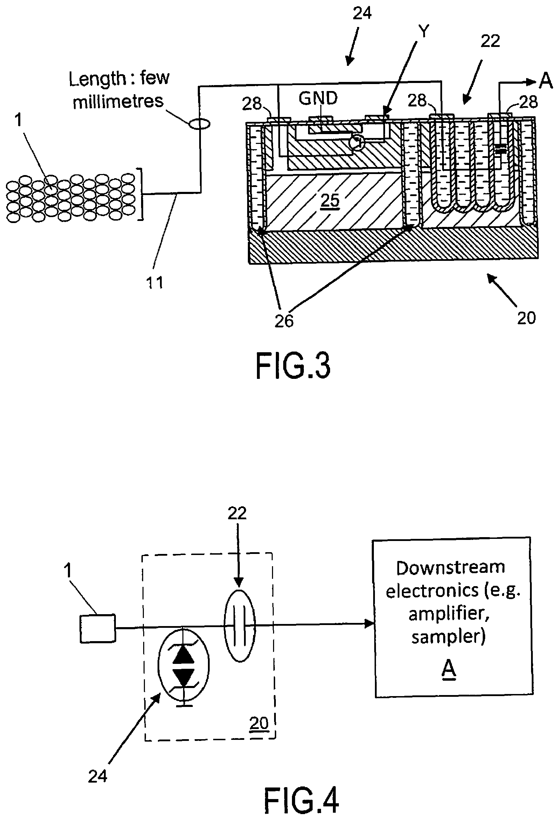

FIG. 3 is a diagram illustrating the structure of a biological-electrode protection module according to a first embodiment of the invention;
FIG. 4 is a diagram illustrating an equivalent circuit of the biological-electrode protection module of Fig.3 connected to a downstream ASIC;

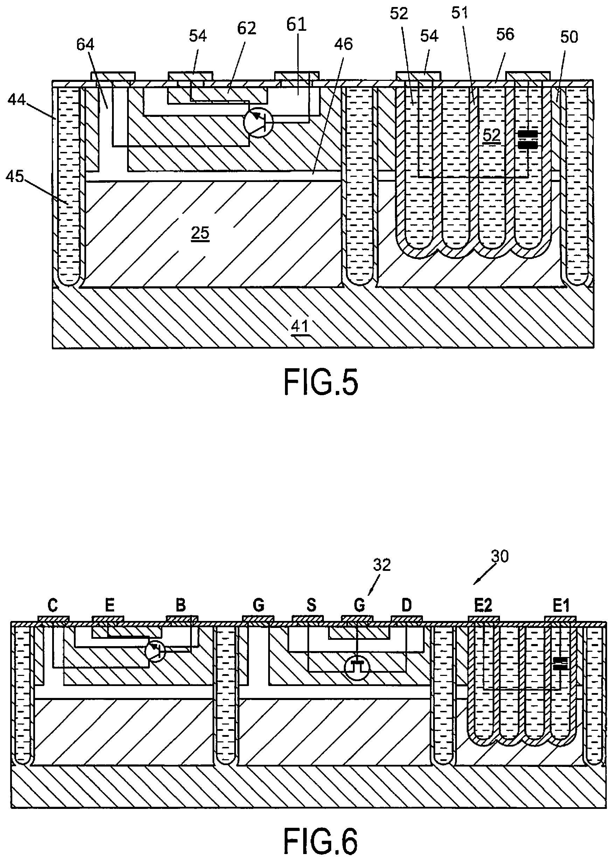

FIG. 5 is an enlarged representation of the biological-electrode protection module of Fig.3;
FIG. 6 is a diagram illustrating the structure of a biological-electrode protection module according to a second embodiment of the invention;

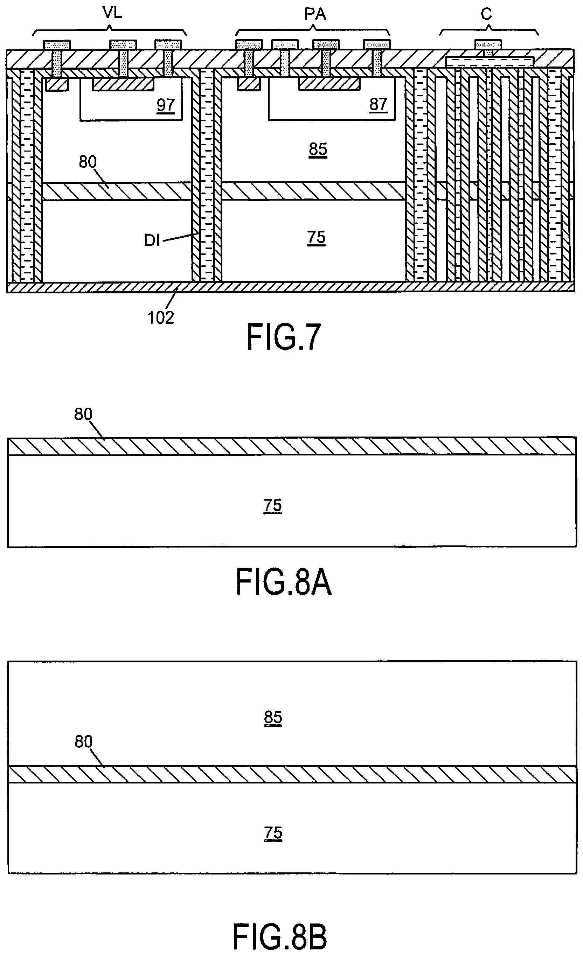

FIG. 7 is a diagram illustrating the structure of a biological-electrode protection module according to a third embodiment of the invention;
FIGs. 8A to 8H are a series of views to illustrate stages in an example method of manufacturing the module of Fig.7; and

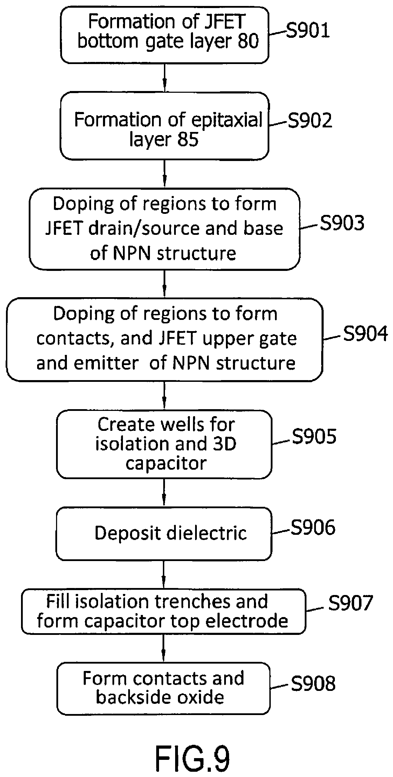

FIG.9 is a flow diagram of the manufacturing method of FIGs. 8A-8H.

### Detailed Description of Example Embodiments

Embodiments of the present invention provide biological-electrode protection modules to provide electrical protection during electrical sensing and/or electrical stimulation practiced on the human or animal body. Principles of the present invention will become clear from the following description of certain example embodiments. The example embodiments describe functionality occurring during electrical sensing but the skilled person will readily understand that biological-electrode protection modules embodying the invention may also be applied in electrical stimulation systems or in systems which implement both biological sensing and stimulation.

Fig.2A and 2B illustrates the approach taken in the present invention, which is to provide a monolithic protection module that incorporates a capacitance component and a low-breakdown-voltage voltage-limiting component in a common substrate. The resultant protection module is compact. The surface area of a protection module according to the invention can be around 60 mm² and the thickness of the protection module 20 can be very low (e.g. 150 µm).

Accordingly, as illustrated in Fig.2A, the protection module can be a discrete component 20 that can be positioned very close to the biological electrodes 1, notably less than 1cm away. Indeed, typically, the biological-electrode protection module according to the invention can be positioned so close to the electrodes that the length of the connection wire between a given electrode and a terminal of the protection module can be less than 1mm. Alternatively, as illustrated in Fig.2B, the compact protection device can be implemented as a composite device 20a which integrates the protection module with the electrodes (for example, the biological electrodes are formed directly on top of the die). The heavy border illustrated in Fig.2B indicates schematically a housing for the biological-electrode protection module. In certain embodiments of the invention the housing is made of biocompatible material, e.g. parylene or similar polymers or ceramics, notably so that the module is well-suited to being implanted.

Furthermore, in some embodiments the protection module 30/30a has a pre-amplifier component integrated into the same substrate as the capacitor component and low-breakdown-voltage voltage-limiting component. In such embodiments, an advantage of implementing direct amplification close to the sensing electrode is that the electrical signal output from the protection module 30/30a towards the rest of the signal acquisition electronics has a level which provides better immunity against noise/unwanted parasitic signals. Accordingly, conventional off-the-shelf amplifiers and samplers can be used in the downstream portion of the signal acquisition chain. So, compared to the configurations illustrated in Figs.1A to 1E, the specialized amplifier and filter components in module 3 can be omitted.

The structure of a first embodiment of a discrete biological-electrode protection module 20 according to the invention is illustrated in a simplified manner in Fig.3.

The biological-electrode protection module (20) of this embodiment comprises a capacitor component (22) and a voltage-limiting component (24) integrated in a common substrate (25). Input and output terminals (28) are also provided for interconnection of the biological-electrode protection module 20 to the set of electrodes 1 and to the downstream signal acquisition electronics A.

Fig.4 is a diagram illustrating an equivalent circuit to the configuration illustrated in Fig.3.

As illustrated in Fig.3, only a single voltage-limiting component 24 and capacitor component 22 can be seen in the biological-electrode protection module 20, but it is to be understood that multiple components may be provided depending on the application (and, in particular, depending on the connections that are to be made to the set of biological electrodes).

The number of input/output terminals of the biological-electrode protection module 20 depends on the application and, in particular, on the number of biological electrodes, whether they are operated for sensing or for stimulation or for both (e.g. with individual channels implementing sensing or stimulation in a time-division manner, or with sensing and stimulation performed simultaneously via different channels). In general, the protection module 20 is customized to the specific set of electrodes 1.

The capacitor component 22 used in the biological-electrode protection module 20 is advantageously implemented as a high-density capacitive element. In the example illustrated in Fig.3, the capacitor component is implemented as a three-dimensional capacitor, notably a capacitor having electrode and dielectric layers formed conformally over relief features in the substrate 25. In the illustrated example the 3D capacitor 24 is formed in wells in the substrate 25, but other 3D capacitor structures may be used, for example the electrode and dielectric layers may be formed conformally over pillars/columns in the substrate. Use of 3D capacitors allows a relatively high capacitance value to be achieved in a small space. The high-density capacitors function as DC-blocking elements to prevent any continuous polarization being applied to the patient's body.

The voltage-limiting component 24 used in the biological-electrode protection module 20 is a low-breakdown-voltage voltage-limiting component, notably having a breakdown voltage equal to or less than 6 volts. In the example illustrated in Fig.3, the voltage-limiting component 24 is implemented by exploiting an NPN or PNP transistor-type structure, employed here for its ability to block voltage pulses of both polarities (i.e. equating to a pair of back-to-back diodes which operate in a punch-through mode).

An advantage of implementing the voltage-limiting component 24 as an integrated component having an NPN or PNP structure is the ability to achieve a low voltage voltage-limiter using the punch through mode. This specific voltage-limiting structure has a low breakdown voltage (<3.6V) and can handle large surge current (biphasic pulses), making it particularly well adapted for use in a biological electrode protection module. Moreover, the technology and manufacturing processes needed to implement the PNP or NPN structure is compatible with the technology and manufacturing processes needed to implement the capacitor component, especially in the case of fabricating the capacitor component as one or more integrated 3D capacitors.

The voltage-limiting component 24 may be fabricated to have a particularly low breakdown voltage, e.g. equal to or less than 3.3 volts, so as to make the overall module 20 suitable for use as an implantable device. Voltage-limiting components having still lower breakdown voltages (e.g. equal to or less than 2.2 volts; equal to or less than 1.8 volts; etc.) may also be employed, depending upon the application in which the biological electrodes are used, i.e. in a pacemaker, in neurostimulation, etc. As the operating voltage is reduced the power consumption reduces and this, in turn, may extend the useful life of the product.

In the example illustrated in Fig.3 deep isolation trenches 26 are provided in the substrate 25 extending substantially all the way through the substrate 25. These isolation trenches function to electrically isolate from one another the active and passive components that are integrated into the common substrate 25. Various techniques may be used to form and fill the isolation trenches. The Applicant's earlier patent application EP 18 306 164.7 describes certain isolation trenches and methods to manufacture the trenches. The properties and fabrication steps described in that application can be applied in the present embodiment, and the teaching of that document is incorporated herein by reference. Using the isolation structure proposed in application EP 18 306 164.7 makes it possible to provide high isolation between each channel, which may allow stimulation and sensing to be performed in the same operating phase.

In view of the description in the present document of the structure and function of biological-electrode protection modules embodying the invention and, in particular, the disclosure of which components and component technologies can be integrated together in a common substrate, the skilled person will readily understand how to construct biological-electrode protection modules embodying the principles described herein. Accordingly, the components will not be further described individually in detail.

The invention is not particularly limited having regard to the choice of materials and layer thicknesses in the components illustrated in Fig.3 and the skilled reader will readily understand that these parameters may be varied while still constructing an integrated device according to the invention. Purely for the purposes of illustration, some example materials and values are provided below in relation to the enlarged view illustrated in Fig.5.

In the example illustrated in Fig.5, the substrate 25 is a SOI (silicon on insulator) substrate provided on a multi-layer base consisting of an oxide layer 42 for example silicon oxide (SiO₂). The substrate 25 has a high resistivity, typically >> 4 Ohms.cm, more particularity > 100 Ohms.cm even more particularity between 1K to 3K Ohm.cm), achieved by doping to a corresponding doping concentration, e.g. of 10e14 atoms/cm³ (that is, 10¹⁴ atoms/cm³). A doped region 46 is provided in the substrate 25, and an epitaxial layer 50 is formed above the doped region 46 in the zone where the capacitor component is provided. The thickness of the epitaxial layer 50 is typically of the order of 500 nm to 2 µm. In the capacitor component 22 illustrated in Fig.5, a dielectric layer 51 is formed conformally over wells in the doped region 50 and the substrate 25. As an example, the dielectric layer may advantageously be formed of a material having a relatively high dielectric constant such as silicon nitride, alumina (Al₂O₃), hafnium oxide, etc., and plural layers may be overlain to form the dielectric layer of the capacitor component. The remaining space in the wells is then filled with a conductive material 52 to serve as the capacitor top electrode. The conductive material may, for example, be formed of doped polysilicon, TiN, TaN, NiB, Ru, etc. Terminals 54 made of a conductive material are provided connected to the capacitor electrodes. The conductive material used for the terminals may be, for example, a metal such as Al, Cu, Ag, combined or not with barrier metals such as, for example, TiN or TaN, or made of other metals or alloys, a multi-layer structure containing plural metals and/or alloys, etc.

In the example illustrated in Fig.5, the voltage-limiting component 24 is a punch-through bipolar structure that comprises an n-type region 50 having relatively low doping (typically in the range 10¹³ atoms/cm³ to 10¹⁵ atoms/cm³), a p-type region 61, i.e. a region of opposite polarity from the region 50, typically having a doping level in the range 10¹⁴ atoms/cm³ to 10¹⁶ atoms/cm³, and an n-type region 62 of the same polarity as the region 50 but considerably more heavily doped (typically in the range 10¹⁶ atoms/cm³ to 10²⁰ atoms/cm³). This structure provides back-to-back NP and PN junctions. However, the present invention is not limited to that case; the polarity of each of the regions 50, 61 and 62 can be inverted so that the structure comprises back-to-back NP and PN junctions. Typically, the region 62 has a thickness in the range from 30 to 300 nm, and the layer 61 has a thickness in the range of 30 nm to 100 nm. A via filled with conductive material 64 provides a connection to one end of the voltage-limiting component and a conductive layer 56 provides a connection to the other end of the voltage-limiting component.

In the example illustrated in Fig.5, the 3D capacitor 22 is formed in wells of a depth set in the range from about 50 µm to about 100 µm. Typically, the critical dimension of the well mouth (i.e. the diameter for a well of circular cross-section, or the narrow dimension for a well having an elongated cross-section) is of the order of 1 µm. Typically, the thickness of the dielectric layer is between 1 nm to 100 nm more specifically between 5 and 40 nm.

The skilled person will understand that the material, doping levels, thicknesses, etc. quoted above in regard to the example of Fig5 are purely illustrative and are not limitative of the present embodiment.

Fig.6 illustrates a second embodiment of biological-electrode protection device 30 according to the present invention. This embodiment incorporates a pre-amplifier 32 integrated into the same substrate as the voltage-limiting component and capacitor component. As mentioned above, when using biological-electrode protection modules 30 of this type, that include a pre-amplifier 32, conventional devices for amplification and sampling can be used in the signal acquisition stream downstream, it is not necessary to use advanced amplifiers or special filtering.

In the example illustrated in Fig.6 the pre-amplifier is implemented as a JFET. This presents a number of advantages, notably in terms of simplifying the process required to fabricate the overall product. In particular, because of overlap in technologies:
∘ Common process steps can be used to form the JFET and to form the low voltage voltage-limiting component,
∘ Common process steps can be used to form the deep isolation trenches and the 3D capacitor structures, and
∘ Common process steps can be used to deposit a layer to form the dielectric layer of the capacitor and a layer in the JFET device (to form a MOSFET).

These overlaps in technology keep the fabrication process simple and fast, and may allow the use of just only 2 levels of interconnections.

In the embodiments illustrated in Figs.3-6 the substrate is a SoI substrate, but the invention is not limited to use of such a substrate. In a similar way, in the example illustrated in Figs.3-6 the wells containing the layers of the 3D capacitor are shallower than the wells containing the deep trench isolation, but the invention is not limited to this configuration.

Fig.7 illustrates a third embodiment of biological-electrode protection module according to the present invention. In the embodiment illustrated in Fig.7, similarly to the second embodiment, a preamplifier component PA implemented as a JFET is provided as well as a voltage-limiting component VL in the form of an NPN structure, and a 3D capacitor C. Deep isolation trenches DI are also included in the architecture. However, in this embodiment the deep isolation trenches and the 3D capacitor wells all extend through the entire thickness of the substrate. Moreover, the substrate is a simple doped silicon substrate 75 rather than a SoI substrate, and a backside oxide layer 102 is provided on the rear of the substrate 75.

The skilled person will readily appreciate the process steps that may be used to fabricate a biological-electrode protection module having the voltage-limiting component, capacitor and, optionally, pre-amplifier components discussed above. Nevertheless, for the purposes of illustration, not limitation, a typical process flow for manufacturing the module illustrated in Fig.7 will be described below with reference to Figs.8A-8H and Fig.9.

In a first step S901 antimony is implanted into a P-type Si substrate having a resistivity of 1 kOhm.cm, to form a layer 80 which will constitute a bottom gate of the JFET constituting the preamplifier, as illustrated in Fig.8A.

Next, in a step S902, an epitaxial layer 85 is formed on the layer 80, as shown in Fig.8B. This layer 85 is doped with As.

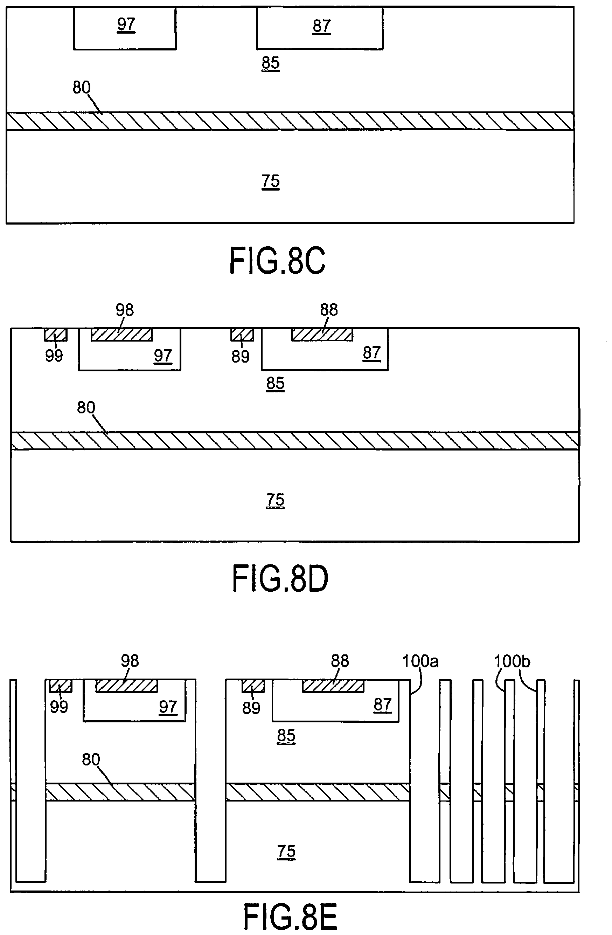

Next, boron is implanted into regions 87 and 97 which will form, respectively, the drain/source of the JFET and the base of the NPN structure, as shown in Fig.8C.

Next, in a doping process S904, As is implanted into regions 88 and 98 which will form, respectively, the upper gate of the JFET and the emitter of the NPN structure, and P or B is implanted into regions 89 and 99 which will form contacts, as shown in Fig.8D.

A common patterning and etching process S905 forms relatively broad wells 100a for use in creating the deep isolation trenches and somewhat narrower wells 100b for use in forming the 3D capacitor, as shown in Fig.8E.

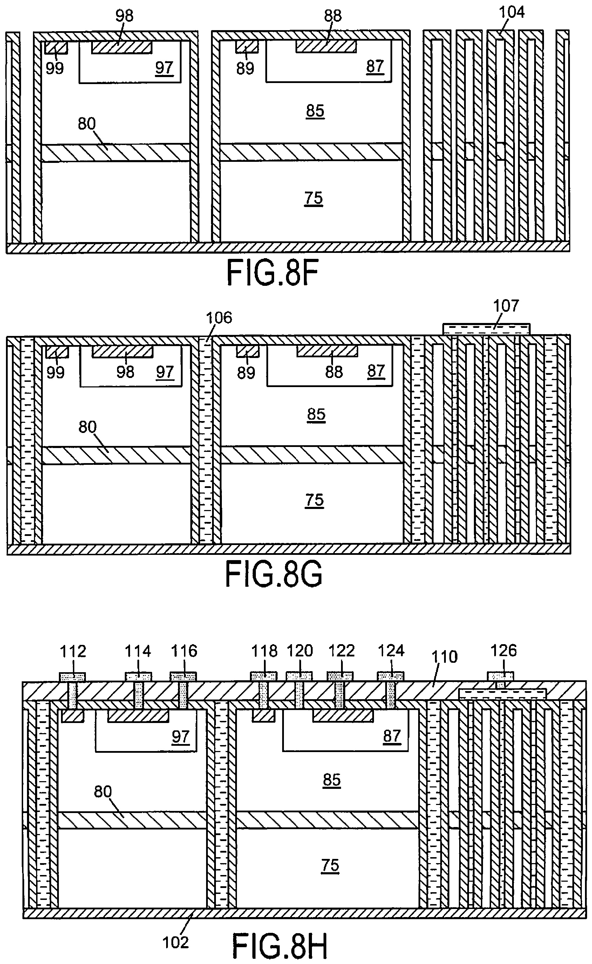

A common deposition process S906 deposits a dielectric layer 104 along the walls of the openings 100a and 100b, as illustrated in Fig.8F. Then, a common deposition process S907 deposits a conductive material into the openings 100a and 100b to constitute filling 106 for the isolation trenches and a top electrode 107 for the 3D capacitor, as illustrated in Fig.8G.

Next, in a stage S908 an insulator layer 110 is deposited on the top of the structure, patterning and deposition processes are implemented to form contacts 112-126 at the top of the module, and a backside oxide layer 102 is formed at the rear of the substrate 75, as illustrated in Fig.8H. The contact 112 connects to the collector of the voltage-limiting NPN structure. The contact 114 connects to the emitter of the voltage-limiting NPN structure. The contact 116 connects to the base of the voltage-limiting NPN structure. The contact 118 connects to bottom gate of the JFET. The contact 120 connects to the source of the JFET. The contact 122 connects to the upper base of the JFET. The contact 124 connects to the drain of the JFET. The contact 126 connects to the top electrode of the 3D capacitor. The bottom electrode of the capacitor is not shown in the figures and may be implemented in different manners, as known by the skilled person, depending on whether it is desired to make contact to the capacitor top and bottom electrodes at the same side of the substrate (e.g. at the top) or at opposite sides of the substrate.

It will be understood that the above description is merely illustrative and numerous aspects of the manufacturing process may be varied. However, the above description is given to illustrate the fact that, when manufacturing a module of the types illustrated in Figs.6 and 7, which combine a preamplifier implemented as a JFET, a voltage-limiting component implemented using an NPN structure (or PNP structure) and a capacitor which is implemented as a 3D capacitor, common processes may be used to form aspects of more than one of the voltage-limiting component, pre-amplifier and 3D capacitor.

As mentioned above, because the protection modules according to embodiments of the invention are particularly compact, they can be laid down on the biological electrodes, or even integrated with the biological electrodes, for example by forming the biological electrodes on the top of the die. Thus, it is feasible to construct implantable devices incorporating biological-electrode protection modules according to certain embodiments of the invention.

Embodiments of the present invention may provide one or more of the following advantages:
- the same Si die can carry high-value capacitors, voltage-limiting structure specifically adapted to handle biphasic pulses and operating in the punch through mode (for low voltage) and thereby avoid the use of oversized active parts;
- because of overlap in technologies used to implement the components, common processes can be shared and used in forming the voltage-limiting component, capacitor component, isolation trenches and pre-amplifier, thus rendering manufacture of the biological-electrode protection module simpler and cheaper;
- the biological-electrode protection module has a high level of integration, producing a compact device capable of being laid down on the biological electrodes;
- the biological-electrode protection module can be customized to connect to multiple sensing/stimulation channels, enabling it to be used with a microelectrode array
- in some embodiments, a bipolar transistor structure (implementing the voltage-limiting component), JFET transistor, deep isolation trenches, and deep-trench capacitors can be integrated in a common substrate (e.g. SOI) to produce a particularly compact structure
- the biological-electrode protection module is compatible with voltage-drive and current-drive architectures
- parasitics are reduced
- direct signal sampling is enabled (no need to shield, to filter, or use a differential approach in the signal acquisition chain downstream of the protection module)
- component matching between channels can be below 0.1%, providing high CMRR (> 70dB).

### Additional Variants

Although the present invention has been described above with reference to certain specific embodiments, it will be understood that the invention is not limited by the particularities of the specific embodiments. Numerous variations, modifications and developments may be made in the above-described embodiments within the scope of the appended claims.

## Claims

1. A biological-electrode protection module (20,30), comprising:
a capacitor component (22) and a voltage-limiting component (24) integrated in a common substrate (25); and
input and output terminals;
wherein the voltage-limiting component has a breakdown voltage equal to or less than 6 volts; and
one of the input and output terminals is configured to connect to each of a set of one or more biological electrodes.

2. A biological-electrode protection module (20,30) according to claim 1, wherein the voltage-limiting component (24) is a biphasic device.

3. A biological-electrode protection module (30) according to any one of claims 1 to 3, further comprising a pre-amplifier component (32) integrated in said substrate (25).

4. A biological-electrode protection module (20,30) according to claim 3, wherein the preamplifier component (32) is a junction field effect transistor.

5. A biological-electrode protection module (20,30) according to any one of claims 1 to 4, connected to said set of biological electrodes (1), wherein there is a distance equal to or less than 1cm between the biological-electrode protection module (20,30) and the set of biological electrodes (1)

6. The biological-electrode protection module (20,30) according to any one of claims 1 to 4, integrated with said set of biological electrodes (1).

7. The biological-electrode protection module (20,30) according to any one of claims 1 to 6, wherein the voltage-limiting component has a breakdown voltage equal to or less than 3.3 volts and the biological-electrode protection module (20,30) is configured to be implanted in a human or animal body.

8. The biological-electrode protection module (20,30) according to any one of claims 1 to 7, wherein the voltage-limiting component (24) comprises an NPN or PNP structure and operates in a punch-through mode.

9. The biological-electrode protection module (20,30) according to any one of claims 1 to 8, wherein the capacitor component comprises one or more three-dimensional capacitors.

10. The biological-electrode protection module (20,30) according to claim 9, wherein one or more isolation trenches (26) comprising electrically-insulating material are provided in the substrate and electrically-isolate the one or more three-dimensional capacitors (22) and the voltage-limiting component (24).

11. A biological implant comprising a biological protection module (20,30) according to any one of claims 1 to 10.

12. A method of fabricating a biological-electrode protection module (20,30), comprising:
forming a capacitor component (22) and a voltage-limiting component (24) in a common substrate (25); and
forming input and output terminals of the biological-electrode protection module (20,30);
wherein the voltage-limiting component (24) has a breakdown voltage equal to or less than 6 volts; and
one of the input and output terminals is configured to connect to each of a set of one or more biological electrodes.

13. A fabrication method according to claim 12, comprising:
forming a pre-amplifier component (32) in the substrate (25);
wherein common masking and doping steps are used in forming the voltage-limiting component (24) and pre-amplifier component (32).

14. A fabrication method according to claim 12 or 13, comprising:
a common etching process forming relief features in the substrate and forming in the substrate one or more isolation trenches interposed between passive components in the substrate; and
providing electrically-insulating material in the isolation trench(es);
wherein the forming of the capacitor comprises forming one or more three-dimensional capacitor including layers formed conformally over said relief features in the substrate.

15. A fabrication method according to claim 13, wherein:
the forming of the voltage-limiting component comprises forming a bipolar structure that operates in punch-through mode;
the forming of the pre-amplifier component (32) comprises forming a junction field effect transistor; and
a common set of process steps forms the voltage-limiting component, capacitor component and pre-amplifier component in the common substrate.
